# EUROPEAN PATENT APPLICATION

(11) **EP 2 813 846 A2**
(43) Date of publication of application: **17.12.2014**
(21) Application number: 14162694.5
(22) Date of filing: 31.03.2014
(51) Int. Cl.: G01N 29/032, A61M 5/36, A61M 1/36

(54) **Segmented ultrasonic transducer and gas bubble sensing device comprising the same**

(30) Priority: 02.04.2013 US 201361807475 P
(71) Applicant: Sonotec Ultraschallsensorik Halle GmbH, 06112 Halle / Saale (DE)
(72) Inventor: Fritsche, Tobias, 06114 Halle/S. (DE); Krause, Werner, 06114 Halle/S. (DE); Bader, Nicki, 06120 Halle/S. (DE); zur Horst-Meyer, Santer Dr., 06120 Halle/Saale (DE); Münch, Hans-Joachim, 06130 Halle/Saale (DE)
(74) Representative: Gulde & Partner

(57) **Abstract**

The invention relates to a gas bubble sensing device (1) with two ultrasonic transducers (2, 3, 8, 9). In order to be able to detect gas bubbles with different sizes reliably, at least one of the ultrasonic transducers (2, 3) comprises a plurality of segments (2', 3') that can be operated independent of each other.

## Description

The invention relates to an ultrasonic transducer. Furthermore, the invention relates to a gas bubble sensing device for sensing gas bubbles in a liquid, with two ultrasonic transducers, between which a flow through channel for a liquid extends.

Ultrasonic transducers and gas bubble sensing devices with ultrasonic transducers are known in the art. For instance, known gas bubble sensing devices are used in intensive-care medicine, in order to assure that blood transported by a life support machine and returned to a patient is free of in admissible gas bubbles, which would be life-threatening for the patient. Furthermore, the gas bubble sensing device is used for a monitoring that no inadmissible gas bubbles are transported with a liquid that shall be dispensed, for instance liquid adhesives or other liquids.

In order to detect gas bubbles in the liquid, one of the transducers emits ultrasound. The other one of the transducers receives the ultrasound, which has past the flow through channel and the liquid flowing there through. In case the liquid comprises a gas bubble, the conduction of the ultrasound is affected by the gas bubble, such that the intensity of the ultrasound received by the second transducer is reduced by the gas bubble compared to a gas bubble free liquid.

Known gas bubble sensing devices with known ultrasonic transducers, however, cannot readily be adapted to detect different sizes of gas bubbles, as the detectable gas bubble size depends from the size of the transducers. Hence, in case different gas bubble sizes shall be reliably detected, different gas bubble sensing devices have to be used. For instance, in intensive care, larger gas bubbles are allowed for adult patients compared to children.

Thus, it is an object of the invention to provide an ultrasonic transducer and a gas bubble sensing device, with which gas bubbles with a wide variety of sizes can be reliably detected without the need to provide more than gas bubble sensing device.

For the ultrasonic transducer mentioned above, the object is achieved in that the ultrasonic transducer comprises a plurality of segments that can be operated independent of each other. For the gas bubble sensing device mentioned above, the object is achieved in that at least one of the ultrasonic transducers is an ultrasonic transducer according to the invention.

When large size gas bubbles shall be detected in the liquid flowing between the transducers, the gas bubbles having a size such that it covers more than one of the segments, the gas bubble can be detected with each of the segments separately or with selected or all of the segments operated as one common segment. In particular, the large size gas bubble can be detected by all of the segments together. However, in case the gas bubble is smaller, the ultrasound conduction through the liquid is not sufficiently reduced in order to achieve a corresponding signal from the other one of the transducers. Yet, in case only selected segments or only one segment is operated independent of the other segments, the size of the operated part of the transducer is reduced, such that the small size gas bubble covers a larger percentage of the operated segment or segments than of the complete ultrasonic transducer. Thus, the ultrasound conduction reduction detected by the operated segment or segments is sufficient in order to generate a signal that reliably indicates a small gas bubble.

Small gas bubbles may have a size comparable, less or slightly larger to a size of one or of a group of selected segments in a first, e.g. longitudinal direction. Big gas bubbles may have a size comparable, less or slightly larger of one of the transducers, for instance in a second direction perpendicular to the first direction and parallel to the transducer.

Dependent on the size of the segments and of the amount of segments operated together, a size level, at which gas bubbles of a certain size can be reliably detected, can be easily adjusted.

The solutions according to the invention can be combined as desired and further improved by the further following embodiments that are advantages on their own, in each case and if not stated to the contrary.

According to a first possible embodiment of the ultrasonic transducer, the segments can be arranged after each other in a longitudinal direction of the ultrasonic transducer. In particular, the ultrasonic transducer is larger in the longitudinal direction than in another direction, such that a large amount of segments, for example three, four, five, up to ten or more, can be provided. Providing an amount of segments as large as possible allows for an exact adjustment of the size of the gas bubbles that can be detected. Further size differences of gas bubbles can be detected by the gas bubble sensing device.

When mounted to the gas bubble sensing device, the segments can be arranged after each other in a longitudinal direction of the flow through channel, such that the fluid flows along all of the segments. In particular, the fluid may transport gas bubbles along each of the segments, thereby providing that the segments can be operated in desired configurations. The segmented ultrasonic transducer and the flow through channel may thus have the same longitudinal direction.

The segments may all have the same size in the longitudinal direction. Preferably, however, a size of one of the segments differs from a size of another one of the segments, the sizes preferably extending parallel to the longitudinal direction. In particular, all of the segments can have different sizes. By using segments with different sizes, the span of gas bubble sizes that can be detected is increased. For instance, the sizes of at least two, selected or of all of the segments decrease in the longitudinal direction.

The size of one of the segments may correspond to the size of another one of the segments, the other one of the segments being arranged before the one segment in the longitudinal direction and multiplied with a predetermined factor. The factor may be a natural number and for instance two, three, four, five or more. Hence, segments with a large variety of sizes are provided, wherein an easy dependency of the sizes exists that can easily be considered when estimating the gas bubble size from signals received from the segmented transducer.

At least two and in particular all of the segments can be formed by separate transducing elements. Separate transducing elements avoid or at least reduce crosstalk between the segments. However, mounting the at least two and in particular all of the plurality of segments independently as separate transducing elements is complex.

In order to facilitate mounting the gas bubble sensing device, at least two or even all of the segments of the ultrasonic transducer are formed by a common transducing element. The common transducing element can be handled as one piece and can therefore easily be mounted. Crosstalk between the segments is often small enough to avoid a disturbance of the gas bubble detection.

In order to reduce cross talk between the segments of the common transducing element, a groove or a channel may extend through the common transducing element between two adjacent of the segments.

Each of the segments may comprise a contact element that is formed separate of a contact element of another one of the segments in order to be able to electrically contact each of the segments independent of another one of the segments. The contact elements may be metal layers that are all formed on the same side of the transducer. On another side and in particular on an opposite side of the transducer, a supply contact, e.g. another metal layer, may be provided, that can contact all of the segments directly.

Contact elements of adjacent segments are preferably arranged at a distance to each other in the longitudinal direction, such that a direct electrical contact between the adjacent contact elements is avoided.

The transducer may be formed with a contact side for electrically contacting the transducer, wherein each of the contact elements is arranged on the contact side. The contact side preferably faces away from the flow through channel and is readily accessible when assembling the gas bubble sensing device, such that lead wires can be easily attached to the contact elements.

The supply contact may be arranged on a mounting side of the ultrasonic transducer that faces the flow through channel and that may be attached to a wall of the flow through channel. In order to be able to attach a lead wire to the supply contact, the supply contact may extend from the mounting side to the contact side, which it can overlap at least sectionwise. The part of the supply contact that overlaps the contact side can be contacted by the lead wire.

In order to avoid a short circuit, the contact elements and the supply contact are preferably arranged at a distance to each other and do therefore not directly contact each other.

The segments may be separated from each other by gaps between the contact elements of the corresponding segments. In particular, edges of the segments are for instance defined by edges of the contact elements. The size of each of the contact elements may correspond to the size of each of the segments. Hence, a segmented ultrasonic transducer can be easily identified and distinguished from an ultrasonic transducer that is not segmented.

The gas bubble sensing device can comprise a signal processing device that may selectively be connected to selected or to all of the segments of the ultrasonic transducer in a signal transmitting manner. The signal processing device is preferably adapted to selectively exchange a separate signal with one of the segments or a common signal with at least two, selected or all of the segments. The signal processing device may provide a signal that causes the ultrasonic transducer and in particular at least one, two, selected or all of the segments to emit ultrasound. Alternatively or additionally, the signal processing device may be configured to receive a measurement signal generated by one, two, selected or all of the plurality of segments in response to receipt of ultrasound transmitted through the flow through channel. By adjusting the signal processing device, the size sensitivity of the gas bubble sensing device can easily be adjusted.

For instance, the signal processing device comprises at least one multiplex element, which contact one, two, selected or all of the plurality of segments in a signal transmitting manner.

The invention is described herein after in greater detail and in an exemplary manner using advantageous embodiments and with reference to the drawings. The described embodiments are only possible configurations, in which, however, the individual features as described above can be provided or combined independently of one another or can be omitted in the drawings, unless stated otherwise:
Figures 1 to 3 show exemplary embodiments of the gas bubble sensing device according to the invention in cross-sectional views;
Figures 4 and 5 show an embodiment of the ultrasonic transducer according to the invention in different views;
Figures 6 and 7 show another exemplary embodiment of the ultrasonic transducer in different views;
Figures 8 and 9 show a further advantageous embodiment of the ultrasonic transducer according to the invention in different views;
Figure 10 shows an embodiment of the gas bubble sensing device according to the invention in a schematic view.

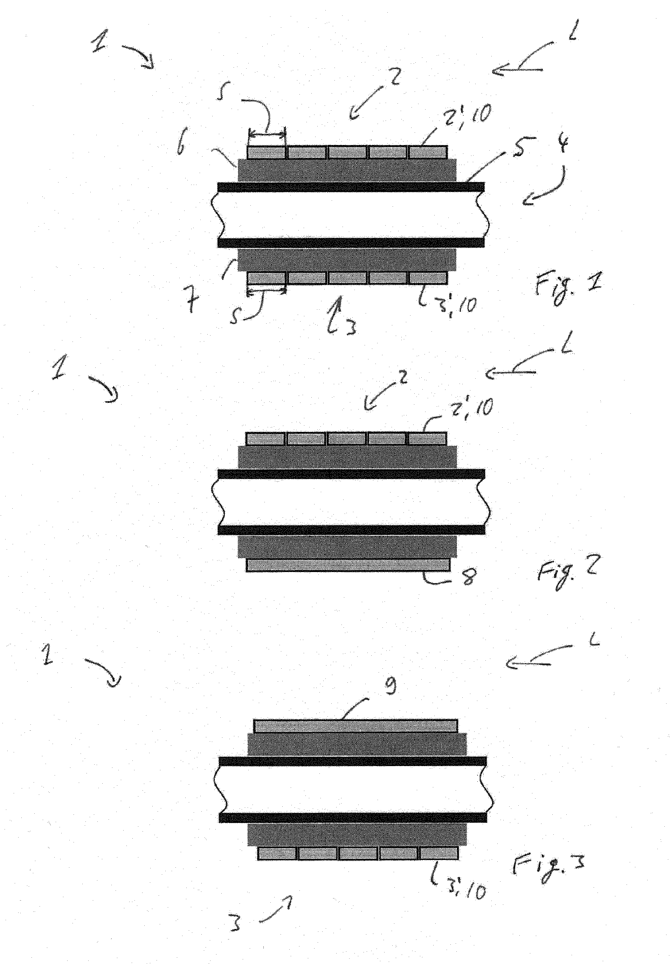

Figure 1 shows a gas bubble sensing device 1 partly in a schematic cross-sectional view, sectioned along a longitudinal direction L according to a first possible embodiment of the invention. The gas bubble sensing device comprises two ultrasonic transducers 2, 3 that extend along the longitudinal direction L. Between the ultrasonic transducers 2, 3, a flow through channel 4 extends parallel to the longitudinal direction L. Through the flow through channel 4, a liquid may flow in the longitudinal direction L. In figure 1, however, a flexible tube 5 is arranged in the flow through channel 4, through which a liquid that shall be checked from gas bubbles may flow. In the flow through channel 4, the tube 5 is elastically deformed and rests against side walls 6, 7 of the flow through channel 4, each of the side walls 6, 7 being arranged between the flow through channel 4 and one of the ultrasonic transducers 2, 3.

During operation of the gas bubble sensing device 1, one of the ultrasonic transducers 2, 3 receives a control signal and emits ultrasound that is conducted perpendicular to the longitudinal direction L towards the other one of the ultrasonic transducers 2, 3 and through the flow through channel 4. A gas bubble free liquid conducts the ultrasound better than a liquid with gas bubbles, as the gas bubbles dampen the ultrasound more than the liquid. The ultrasound received by one of the ultrasonic transducers 2, 3 is converted into an electrical measurement signal, which represents the presence and the size of gas bubbles in the liquid when passing between the ultrasonic transducers 2, 3.

In the exemplary embodiment of figure 1, both of the ultrasonic transducers 2, 3 are formed with a plurality of segments 2', 3'. The segments 2' of the ultrasonic transducer 2 can be operated jointly or independently of each other. Similarly, the segments 3' of the ultrasonic transducer 3 can be operated jointly or independently of each other. As an example, the segments 2', 3' are formed as separate transducing elements 10 in figure 1. The segments 2', 3' of each of the ultrasonic transducers 2, 3 are arranged one after the other in the longitudinal direction L. For instance, the segments 2', 3' of the ultrasonic transducers 2, 3 all have the same size S and for instance the same width parallel to the longitudinal direction L.

Figure 2 shows another embodiment of the gas bubble sensing device 1 in a schematic cross-sectional view. Same reference signs are being used for elements, which correspond in function and/or structure to the elements of the exemplary embodiment of figure 1. For the sake of brevity, only the differences from the exemplary embodiment of figure 1 are looked at.

In figure 2, only the ultrasonic transducer 2 comprises a plurality of segments 2'. Instead of the segmented ultrasonic transducer 3, an ultrasonic transducer 8 is arranged opposite of the ultrasonic transducer 2 that is not segmented, but can only be operated completely, hence in one piece. In the longitudinal direction L, the ultrasonic transducers 2 and 8 may have the same width.

Figure 3 shows another embodiment of the gas bubble sensing device 1 in a schematic cross-sectional view. Same reference signs are being used for elements, which correspond in function and/or structure to the elements of the exemplary embodiments of figures 1 and 2. For the sake of brevity, only the differences from the exemplary embodiments of figures 1 and 2 are looked at.

According to the exemplary embodiment shown in figure 3, the ultrasonic transducer 3 comprises segments 3'. An ultrasonic transducer 9 that is provided opposite of the ultrasonic transducer 3 replaces the segmented transducer 2 and is not segmented, but can rather only be operated completely, hence as one piece.

In the exemplary embodiments of figures 1, 2, 3, the segments 2', 3' are exemplarily formed as separate transducing elements 10. The ultrasonic transducers 2 or 9 may be operated to emit ultrasound in response to a control signal. The ultrasonic transducers 3 or 8 may be operated to receive ultrasound conducted through the flow through channel 4 and emitted from the other ultrasonic transducers 2 or 9 and to convert the received ultrasound into a measurement signal that is representative for bubbles in the liquid. Hence, either emitting ultrasonic transducers 2, 9 or receiving ultrasonic transducers 3, 8 or even both ultrasonic transducers 2, 3, 8, 9 may be segmented, wherein the segments can be operated independent of each other.

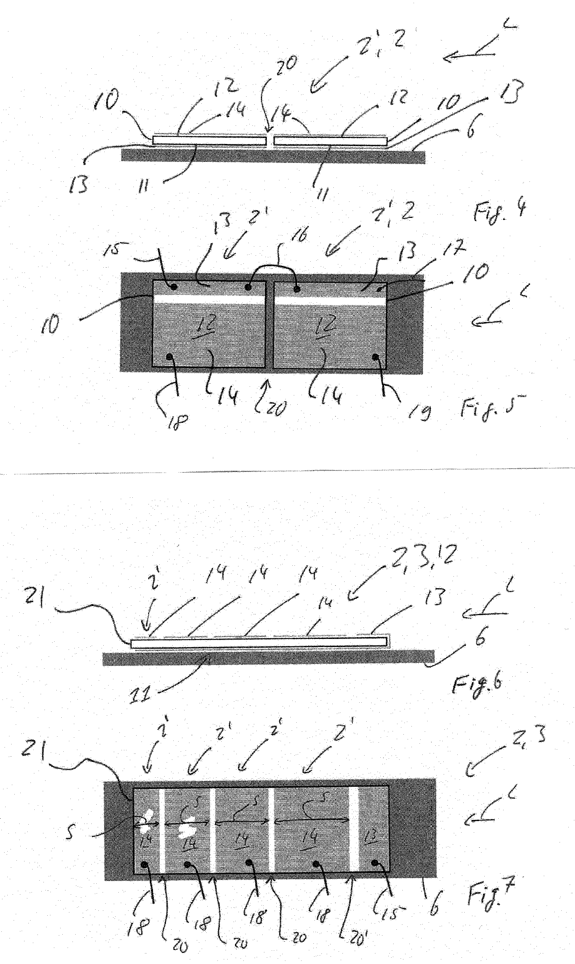

Figures 4 and 5 show two adjacent segments 2', 3' of one of the segmented ultrasonic transducers 2, 3 of the exemplary embodiments 1, 2, 3 in an enlarged elevation. For the sake of brevity, only segments with the reference numeral 2' are described in the following. However, segments with reference numeral 3' may have an identical structure as described in the following.

Each of the segments 2' comprises one of the separate transducing elements 10. The ultrasonic transducer 2 is formed with a mounting side 11, which faces the flow through channel 4 and which is in contact with one of the side walls 6, 7. In the exemplary embodiment of figures 4 and 5, merely side wall 6 is shown.

The mounting side 11 may be affixed to the side wall, e. g. by gluing, such that ultrasound generated by the ultrasonic transducer 2 is effectively transmitted to the side wall 6 or such that ultrasound is effectively conducted to the ultrasonic transducer 3. Opposite of the mounting side 11, the ultrasonic transducer 2 is formed with a contact side 12 that faces away from the flow through channel 4.

Between the side wall 6 and the mounting side 11 of each of the segments 2', a supply contact 13 is arranged. The supply contact 13 electrically contacts one of the segments 2'. On the contact side 12 of each of the segments 2', a contact element 14 is provided.

Lead wires can be readily attached to the contact elements 14, as the contact elements 14 face away from the flow through channel 4 and from the side wall 6 when the ultrasonic transducer 2 is mounted. However, the supply contact 13 cannot readily be brought in contact with a lead wire without affecting the ultrasound conductivity between the ultrasonic transducer 2 and the side wall 6. In order to be able to attach a lead wire to the supply contact 13, the supply contact 13 may extend from the mounting side 11 onto the contact side 12, where it can easily be contacted by a lead wire.

As shown in figure 5, a lead wire 15 is attached to the supply contact 13 and in particular to a section of the supply contact 13, which is arranged on the contact side 12. Another lead wire 16 interconnects supply contacts 13 of adjacent segments 2'. Another lead wire 17 may be provided, in case supply contacts 13 of at least one further segment 2' shall be connected to the supply contact 13 of the segment 2' shown. Each of the contact elements 14 may be in contact with a separate lead wire 18, 19.

The lead wires 15 to 19 are adapted to conduct signals to or from the ultrasonic transducer 2.

The segments 2' are arranged at a distance to each other in the longitudinal direction L, such that a gap 20 is arranged between the segments 2'. In the exemplary embodiment of figures 4 and 5, the gap 20 separates the separate transducing elements 10 from each other. Figures 6 and 7 show another exemplary embodiment of the ultrasonic transducers 2, 3 in different schematic views. Same reference signs are being used for elements, which correspond in function and/or structure to the elements of the previous exemplary embodiments. For the sake of brevity, only the differences from the exemplary embodiments of figures 1 to 5 are looked at.

The ultrasonic transducer 2 of the exemplary embodiment of figures 6 and 7 does not comprise separate transducing elements 10, but a common transducing element 21. The common transducing element 21 is formed as one piece, such that the ultrasonic transducer 2 can be mounted more easily than the ultrasonic transducers 2, 3 of the previous embodiments. In order to be able to operate the segments 2' independent of one another, one contact element 14 is provided for each of the segments 2'. The contact elements 14 are arranged on the contact side 12 at a distance to each other in the longitudinal direction L. When operating the ultrasonic transducer 2, electrical signals provided to or from the ultrasonic transducer 2 are limited to one of the segments 2' due to the arrangement of the corresponding contact element 14.

Between each of the contact elements 14, the gap 20 extends perpendicular to the longitudinal direction L, the gap 20 separating the contact elements 14 and the segments 2' in the longitudinal direction L from each other. Furthermore, a gap 20' extends between the contact element 14 of the first segment 2' and the section of the supply contact 13 that is arranged on the contact side 12. The gap 20 between the contact elements 14 separates the segments 2' from each other. Hence, the contact elements 14 define the size and/or the position of the segments 2'

The common transducer element 21 is provided with a single supply contact 13 in the exemplary embodiment of figures 6 and 7, which essentially covers the mounting side 11 of the ultrasonic transducer 2 completely and which extends from the mounting side 11 onto the contact side 12, where it can be contacted by a lead wire.

The size of the segments 2' may be the same for all segments 2'. However, as particularly recognizable in figure 7, the size S of the segments 2' may vary. In particular, the size S may be a width of each of the segments 2', the width extending parallel to the longitudinal direction L. For instance, the size S and in particular the width of the segments 2' can decrease in the longitudinal direction L. For example, the size S of the segments may correspond to a linear or another monotone function. In the embodiment of figure 7, the penultimate segment 2' in the longitudinal direction L has twice the width of the last segments 2'. The segment 2' before the penultimate segments 2' has three times the width of the last segment 2'. The first segment 2' in the longitudinal direction L has four times the width of the last segment 2'.

Each of the contact elements 14 of the segments 2' is contacted by a lead wire 18. The supply contact 13 is contacted by a lead wire 15. All lead wires 15, 18 of figure 7 are separate lead wires.

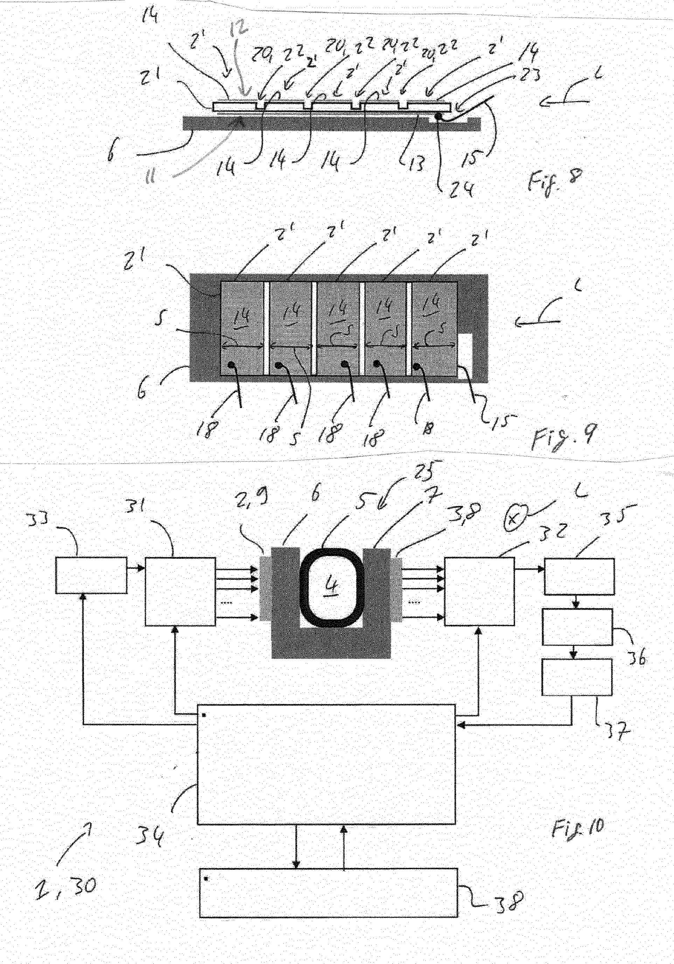

Figures 8 and 9 show another exemplary embodiment of the ultrasonic transducer 2 in different schematic views. Same reference signs are being used for elements, which correspond in function and/or structure to the elements of the previous exemplary embodiments. For the sake of brevity, only the differences from the previous exemplary embodiments are looked at.

Again, only ultrasonic transducer 2 with segments 2' is described in the following. Of course, ultrasonic transducer 3 may correspond in structure and/or function to the ultrasonic transducer 2 shown in figures 6 and 7.

In the exemplary embodiment in figures 8 and 9, the common transducing element 21 is formed with grooves 22, that extend perpendicular to the longitudinal direction L and that open in the contact side 12 away from the side wall 6. Each of the gaps 20 overlaps one of the grooves 22 perpendicular to the longitudinal direction L and away from the side wall 6. By providing the common transducing element 21 with grooves 22 between the segments 2', crosstalk between the segments 2' can be reduced.

In order to increase the size or the amount of the segments 2', the supply contact 13 may not extend onto the contact side 12 of the ultrasonic transducer 2, but may completely be arranged on the mounting side 11, according to the exemplary embodiment of figures 8 and 9. The provision of the grooves 22 is, however, independent of the form of the supply contact 13.

In order to be able to contact the supply contact 13 with the lead wire 15, the side wall 6 is formed with a contact indention 23, via which the lead wire 15 can extend to the supply contact 13. For instance, the lead wire 15 can be soldered or welded to the supply contact 13, wherein a solder or weld joint 24 between the supply contact 13 and the lead wire 15 may at least sectionwise be arranged in the contact indention 23.

As can be seen in figure 9, the size S and in particular the width of the segments 2' is the same for all segments. However, forming segments 2' with identical sizes S is independent of the provision of grooves 24, of the form of the supply contact 13, of the provision of the side wall 6 with the contact indention 23, or of the provision of separate transducing element 10 or a common transducing element 21.

Figure 10 shows another embodiment of the gas bubble sensing device 1 in a schematic frontal elevation. Same reference signs are being used for elements, which correspond in function and/or structure to the elements of the previous exemplary embodiments. For the sake of brevity, only differences from the previous exemplary embodiments are looked at.

The gas bubble sensing device 1 comprises the ultrasonic transducers 2, 3 or the ultrasonic transducers 2, 8 or the ultrasonic transducers 3, 9 of the previous embodiments. The tube 5 is pressed into the flow through channel 4 and extends in the longitudinal direction L, which points into the plane of projection.

Perpendicular to the longitudinal direction L, the ultrasonic transducers are arranged after each other with the flow through channel 4 therebetween. A projection of one of the ultrasonic transducers 2, 3, 8, 9 perpendicular to the longitudinal direction L defines a measurement cell 25 that extends through the flow through channel 4, wherein bubbles can be detected in fluid flowing through the measurement cell 25.

The gas bubble sensing device 1 may furthermore comprise a signal processing device 30 that is connected to each of the ultrasonic transducers 2, 3, 8, 9 in a signal transmitting manner. The signal processing device 30 is adapted to selectively exchange a separate signal with each of the segments 2', 3' and/or one of the ultrasonic transducers 8, 9, or a common signal with at least two of the segments 2', 3'.

In order to be able to exchange signals with the segments 2', 3', the signal processing device 30 may comprise at least one multiplex element 31 that can contact the segments 2', 3' independent of each other or in parallel. In case both of the segmented ultrasonic transducers 2, 3 are provided, the signal processing device 30 is preferably formed with two multiplex elements 31, 32. Furthermore, the signal processing device 30 may comprise an ultrasound source 33 that transmits an ultrasound signal to one of the multiplex elements and for instance to multiplex element 31. The ultrasound source 33 may be connected to a control unit 34 and may receive a control signal from the control unit 34. Furthermore, the control unit 34 can be connected to the multiplex element 31 and optionally also to multiplex element 32 in order to control to which of the segments 2', 3' the ultrasound signal is conducted. Upon receipt of the control signal, the respective segment 2' or segments 2' emit ultrasound.

After passing the flow through channel 4, ultrasound emitted by ultrasonic transducers 2 or 9 is received by ultrasonic transducers 3 or 8, which generate a measurement signal that depends from the intensity of the ultrasound received and which is representative for the presence and the size of a gas bubble in the liquid within the measurement cell 25. In order to be able to use measurement signals of each of the segments 3', the multiplex element 32 may be provided. Multiplex element 32 may be connected to the control unit 34 in a control signal transmitting manner, such that receipt and transfer of a measurement signal of a selected segment 3' or of selected or even of all segments 3' can be controlled. Thus, the measurement signal may be derived from one single segment 3' or may be a measurement signal of selected or even of all segments 3'.

The multiplex element 32 may forward the measurement signal to an amplifier 35 which amplifies the measurement signal. The amplified measurement signal may be forwarded to a peak detector 36 and then to an analog to digital converter 37. From the analog to digital converter 37, the digitized measurement signal may be provided to the control unit 34, which determines the size of gas bubbles in the fluid based on the measurement signal. In case the size of an air bubble is above a predetermined allowable maximum size, the control unit 34 may generate a warning signal, which can be sent to another device 38 that may be a life support machine or an injector or dispenser of fluids. Furthermore, a maximum size signal may be received by the signal processing unit 30, e.g. from the other device 38, the maximum size signal representing the maximum size of an allowable gas bubble or the minimum size of an unallowable gas bubble. Hence, the signal processing unit 30 and for example the control unit 34 may comprise a maximum size input for receiving the maximum size signal.

## Claims

1. Ultrasonic transducer (2, 3), **characterized in that** the ultrasonic transducer (2, 3) comprises a plurality of segments (2', 3') that can be operated independent of each other.

2. Ultrasonic transducer (2, 3) according to claim 1, **characterized in that** the segments (2', 3') are arranged after each other in a longitudinal direction (L) of the ultrasonic transducer (2, 3).

3. Ultrasonic transducer (2, 3) according to claim 1 or 2, **characterized in that** a size (S) of one of the segments (2', 3') differs from a size (S) of another one of the segments (2', 3').

4. Ultrasonic transducer (2, 3) according to claim 3, **characterized in that** the sizes (S) of at least two of the segments (2', 3') decreases in the longitudinal direction (L).

5. Ultrasonic transducer (2, 3) according to claim 3 or 4, **characterized in that** size (S) of one of the segments (2', 3') corresponds to the size (S) of another one of the segments (2', 3') that is arranged before the one segment in the longitudinal direction (L) and multiplied with a predetermined factor.

6. Ultrasonic transducer (2, 3) according to any of claims 1 to 5, characterized that at least two of the segments (2', 3') are formed by separate transducing elements (10).

7. Ultrasonic transducer (2, 3) according to any of claims 1 to 6, **characterized in that** at least two of the segments (2', 3') are formed of a common transducing element (21).

8. Ultrasonic transducer (2, 3) according to claim 7, **characterized in that** a groove (22) extends through the common transducing element (21) between two adjacent of the segments (2', 3').

9. Ultrasonic transducer (2, 3) according to any of claims 1 to 8, **characterized in that** each of the segments (2', 3') comprises a contact element (14) that is formed separate of a contact element (14) of another one of the segments (2', 3').

10. Ultrasonic transducer (2, 3) according to claim 9, **characterized in that** contact elements (14) of adjacent segments (2', 3') are arranged at a distance to each other in the longitudinal direction (L).

11. Ultrasonic transducer (2, 3) according to claim 9 or 10, **characterized in that** the transducer (2, 3) is formed with a contact side (12) for electrically contacting the transducer (2, 3) wherein each of the contact elements (14) is arranged on the contact side (12).

12. Ultrasonic transducer (2, 3) according to claim 9 or 11, **characterized in that** the segments (2', 3') are separated from each other by gaps (20) between the contact elements (14) of the corresponding segments (2', 3').

13. Gas bubble sensing device (1) for sensing gas bubbles in a liquid, with two ultrasonic transducers (2, 3, 8, 9), between which a flow through channel (4) for a liquid extends, **characterized in that** at least one of the ultrasonic transducers (2, 3, 8, 9) is an ultrasonic transducer (2, 3) according to any of claims 1 to 12.

14. Gas bubble sensing device (1) according to claim 13, **characterized in that** the segments (2', 3') are arranged after each other in a longitudinal direction (L) of the flow through channel (4).

15. Gas bubble sensing device (1) according to claim 13 or 14, **characterized by** a signal processing device that is connected to selected of the segments (2', 3') of the ultrasonic transducer (2, 3) in a signal transmitting manner, and that is adapted to selectively exchange a separate signal with one of the segments (2', 3') or a common signal with at least two of the segments (2', 3').
